# EUROPEAN PATENT APPLICATION

(11) **EP 3 904 342 A1**
(43) Date of publication of application: **03.11.2021**
(21) Application number: 20171715.4
(22) Date of filing: 28.04.2020
(51) Int. Cl.: C07D 233/61

(54) **PROCESS FOR THE PREPARATION OF 3-(TRIFLUOROMETHYL)-5-(4-METHYL-1H-IMIDAZOLE-1-YL)-BENZENEAMINE HYDROCHLORIDE**

(71) Applicant: Grindeks, a joint stock company, 1057 Riga (LV)
(72) Inventor: Konosonoks, Armands, LV-1057 Riga (LV)

(57) **Abstract**

The invention provides an environmentally acceptable and cost effective process of preparation 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide intermediate-3-(trifluoromethyl)-5-(4-methyl-1*H*-imidazole-1-yl)-benzeneamine hydrochloride.

## Description

### Technical Field

The present invention provides an environmentally acceptable and more cost effective process of preparation 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide intermediate - 3-(trifluoromethyl)-5-(4-methyl-1*H*-imidazole-1-yl)-benzeneamine hydrochloride of formula I, without additional purification.

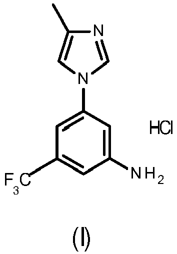

### Background Art

4-methyl-N-[3-(4-methyl-1*H*-imidazol-1-yl)-5-(trifluoromethyl)phenyl]-3-[[4-(3-pyridinyl)-2-pyrimidinyl]amino]-benzamide of formula II 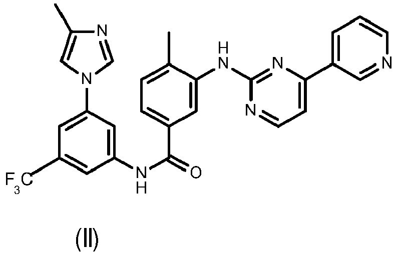 is a tyrosine kinase inhibitor used for the treatment of drug-resistant chronic myelogenous leukemia (CML), and in particular, for the treatment of chronic phase and accelerated phase Philadelphia chromosome positive chronic myeloid leukemia (CML) in adult and paediatric patients whose disease has progressed on or who cannot tolerate other therapies that included imatinib. 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide is administered as a hydrochloride salt in forms of capsules that are marketed in the USA and the EU under the brand name Tasigna®.

The preparation of 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide and the use thereof, especially as an antitumor agent, is described in WO 2004/005281 A (NOVARTIS AG) 04/08/2003.

WO 2006/135640 A (NOVARTIS AG) 07/06/2006 and WO 2006/135619 (NOVARTIS AG) 07/06/2006 describes the preparation of 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide and intermediate of formula (III).

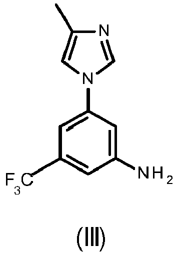

This reaction comprising reacting 4-methyl-1*H*-imidazole with 3-bromo-5-trifluoromethyl-phenylamine in the presence of a transition metal catalyst, such as a copper, a strong to mild base, preferably a carbonate, and optionally a coordinating additive, such as a 1,2-diamine in a dipolar aprotic solvent.

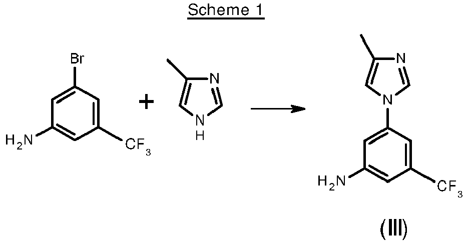

### Summary of invention

The present invention provides a new one-pot process for the manufacture of 3-(trifluoromethyl)-5-(4-methyl-1*H-*imidazole-1-yl)-benzeneamine hydrochloride, using green chemistry principles comprising the step of reacting 3-bromo-5-trifluoromethylaniline with 4-methylimidazole and transformation into hydrochloride salt.

3-(trifluoromethyl)-5-(4-methyl-1 *H*-imidazole-1-yl)-benzeneamine is an intermediate for the preparation of 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide.

Hence, the present invention provides environmentally friendly and more cost effective process for preparing regioisomeric pure 5-(4-methyl-1*H-*imidazol-1-yl)-3-(trifluoromethyl)-benzenamine hydrochloride of formula (I) with high yield and ready to use into next step of preparation 4-methyl-*N-*[3-(4-methyl-1*H*-imidazol-1-yl)-5-(trifluoromethyl)phenyl]-3-[[4-(3-pyridinyl)-2-pyrimidinyl]amino]-benzamide without additional purification. Method comprising reacting 4-methyl-1H-imidazole with 3-bromo-5-trifluoromethyl-phenylamine using a transition metal catalyst, an additional suitable base without solvent with the next transformation into hydrochloride salt of formula (I).

### Brief description of drawings

The general one-pot reaction scheme of the invention can be illustrated in the following embodiments. In a first embodiment, the present invention provides the general process of making compound (I) as follows:

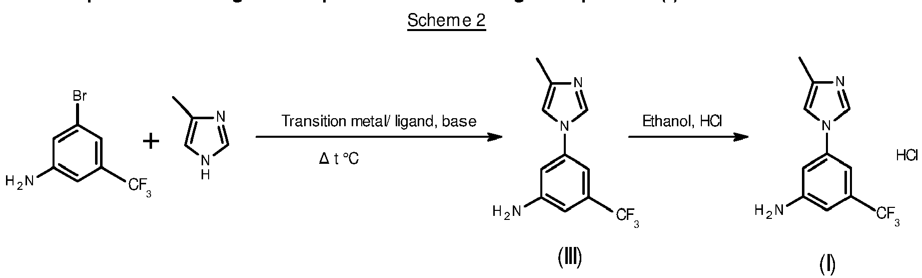

Step I involves 4-methyl-1*H*- imidazole reaction with 3-bromo-5-trifluoromethyl-phenylamine in the presence of a transition metal catalyst and a base for the synthesis of 3-(trifluoromethyl)-5-(4-methyl-1 *H-*imidazole-1-yl)-benzeneamine (II) in absence of solvent. Step II is a preparation hydrochloride salt of compound (II) leading to compound (I).

The metal for transition metal catalyst may be selected from copper or palladium compound, preferably a copper(I) salt, ligand is selected from C-N coupling reactions acceptable ligands such as 1,3-propanediamine, 1,2-dimethylethylenediamine, *N*, *N*, *N*-trimethyl-1,2-ethanediamine, triethylenetetramine, *trans*-1,2-diaminocyclohexane, 8-hydroxyquinoline. The base used in Step I is strong inorganic base, such as potassium carbonate, tripotassium phosphate or caesium carbonate. It has been found, that the coupling reaction of methylimidazole and bromophenylamine have good yield with tripotassium phosphate and caesium carbonate. Tripotassium phosphate is cheaper than caesium carbonate and easy in manufactory use.

3-Bromo-5-trifluoromethyl-phenylamine, 4-methyl-1*H*- imidazole and are in ratio 1:1,2:1,2, less base quantity reduce yield and enhance quantity of unnecessary regioisomeric product. Additionally has been found, in accordance with the present invention that the coupling reaction of 4-methyl-1*H*- imidazole and 3-bromo-5-trifluoromethyl-phenylamine works better in absence of solvent, at a temperature in the range of 160-170°C. Reaction without solvent solved the problem with product stability as disclose in patent EP 1896425 B (NOVARTIS AG) 07/06/2006 in high temperature and in the presence of strong base. Additionally this solution reduces impact on the environment and product costs.

Step II involves the transformation process of compound (II) into a salt of the compound (I), for isolation and purification. Here a solution of compound (II) is treated with an acid in an organic solvent, followed by isolation of the salt (I) by filtration. This transformation is necessary to the product isolation from reaction mass and transformation conditions acceptable to product purification from unnecessary regioisomeric product. After filtration and drying product is ready to use in the next step of 4-methyl-*N*-[3-(4-methyl-1*H*-imidazol-1-yl)-5-(trifluoromethyl)phenyl]-3-[[4-(3-pyridinyl)-2-pyrimidinyl]amino]-benzamide preparation without additional purification.

Furthermore it has been found that Cu(I) with 8-hydroxyquinoline is better transition metal and ligand combination, because this combination is less sensitive for catalyst quantity changes.

The following examples more particularly illustrate the present invention by referring to the following non-limiting examples.

### Description of embodiments

### Example 1

3-Bromo-5-trifluoromethylaniline (15,0 mmol), 4-methylimidazole (18,0 mmol) and caesium carbonate (20,0 mmol) in toluene (30 mL) were stirred under argon 0,5 hours. (2,2'-bis(diphenylphosphino)-1,1'-binaphthyl) (BINAP) (15,00 mmol) and tris(dibenzylideneacetone)dipalladium (0) (15,00 mmol) were added to the mass and heated to 110°C, and keep at this temperature for 36 hours. The hot mass were allowed to cool down to room temperature. The mass was filtered and washed on the filter with toluene, solvent were distillated to 40 mL. The solution was washed with conc. NH₃ and conc. sodium chloride solution. Activated charcoal was added to the solution, the mass brought to boiling, and filtered hot. The filtrate was added toluene to 50 mL and added 50 mL acetone. Intensive stirred the solution was added conc. 3,0 mL HCI dropwise. The reaction mass stirred 0,5 hour and cooled to (0-5)°C. The suspension was filtered and on the filter was rinsed with cold toluene/acetone (1:1) solution, dried. Dry technical product recrystallised from ethanol (35 mL), filtrated and dried at 90 °C under vacuum (0,2-0,3 bar). 0,1 Mmol (0,7 %) of 5-(4-methyl-1H-imidazol-1-yl)-3-(trifluoromethyl)aniline hydrochloride was obtained.

### Example 2-15

5-(4-methyl-1H-imidazol-1-yl)-3-(trifluoromethyl)aniline hydrochloride was obtained in the procedure which carried out as in Example 1, but changed palladium to copper and tried ligands and solvent combinations:

**Table 1**

| Example | Ligands * | Solvent | Temp. °C | Compound (I), yield , % |
|---|---|---|---|---|
| Exaple 2 | DAP | *sec*-Butanol | Boiling point | 1,7 |
| Exaple 3 | DMEDA | *sec*-Butanol | Boiling point | 1,9 |
| Exaple 4 | TrMEDA | *sec*-Butanol | Boiling point | 2,2 |
| Exaple 5 | TMEDA | *sec*-Butanol | Boiling point | 1,7 |
| Exaple 6 | TETA | *sec*-Butanol | Boiling point | 1,5 |
| Exaple 7 | *trans*-DAC | *sec*-Butanol | Boiling point | 1,6 |
| Exaple 8 | DMEDA | Isopropyl alcohol | Boiling point | 0,5 |
| Exaple 9 | DMEDA | Methyl isobutyl ketone (MIBK) | Boiling point | 8,6 |
| Exaple 10 | DMEDA | Toluene | Boiling point | 2,6 |
| Exaple 11 | DMEDA | Acetone | Boiling point | 0,8 |
| Exaple 12 | *trans*-DAC | Methyl isobutyl ketone | Boiling point | 7,6 |
| Exaple 13 | *trans*-DAC | Toluene | Boiling point | 3,1 |
| Exaple 14 | *trans*-DAC | Acetone | Boiling point | 0,8 |
| Exaple 15 | DMEDA | Tetrahydrofuran | Boiling point | 0,2 |

| | | | | |
|---|---|---|---|---|
| * DAP - 1,3-propanediamine, DMEDA - 1,2-dimethylethylenediamine, TrMEDA-N,N,N'-trimethyl-1,2-ethanediamine, TETA - triethylenetetramine, *trans-DAC - trans*-1,2-diaminocyclohexane | | | | |

### Example 16

3-Bromo-5-trifluoromethylaniline (19,6 mmol), 4-methylimidazole (23,6 mmol) and tripotassium phosphate (23,6 mmol) in *n*-butanol (35 mL) were stirred under argon 0,5 hours. 8-Hydroxyquinoline (15,00 mmol) and copper (I) iodide (15,00 mmol) were added to the mass and heated to the *boiling point,* and keep at this temperature for 26 hours. The hot mass was cooled to room temperature. The reaction mixture was filtered and washed on the filter with *n*-butanol, solvent was distillated to 10 mL and added 30 ml of toluene. The solution were washed with conc. NH₃ and conc. sodium chloride solution. Activated charcoal was added to the solution, the mass brought to boiling, and filtered hot. The filtrate was added to stirred 50 mL toluene, 50 mL acetone and conc. 3,5 mL HCI dropwise were added to the solution. The reaction mass was stirred for half an hour and cooledto (0-5)°C. The suspension was filtered and the crude product was washed with cold toluene/acetone (1:1) solution on the filter, dried. Dry technical product recrystallised from ethanol (35 mL), filtrated and dried at 90 °C under vacuum (0,2-0,3 bar). 9,8 Mmol (50 %) of 5-(4-methyl-1*H*-imidazol-1-yl)-3-(trifluoromethyl)aniline hydrochloride was obtained.

### Example 17-19

5-(4-methyl-1H-imidazol-1-yl)-3-(trifluoromethyl)aniline hydrochloride was obtained in the procedure which carried out as in Example 16, but was tried solvent, reaction time and was base quantity:

**Table 2**

| Example | Solvent | Base/Aniline, ratio | Temp., °C | R. time, h | Compound (I), yield % |
|---|---|---|---|---|---|
| Exaple 17 | *n*-BuOH | 2,0:1 | b.p. | 15 | 42,5 |
| Exaple 18 | *n*-BuOH | 2,0:1 | b.p. | 22 | 50,1 |
| Exaple 19 | *n*-BuOH | 1,5:1 | b.p. | 22 | 50,2 |
| Exaple 20 | *n*-BuOH | 1,4:1 | b.p. | 22 | 50,2 |
| Exaple 21 | *n*-BuOH | 1,3:1 | b.p. | 22 | 50,2 |
| Exaple 22 | *n*-BuOH | 1,2:1 | b.p. | 22 | 50,2 |
| Exaple 23 | *n*-BuOH | 1,1:1 | b.p. | 22 | 47,3 |
| Exaple 24 | MIBK | 2,0:1 | b.p. | 15 | 12,7 |
| Exaple 25 | MIBK | 2,0:1 | b.p. | 22 | 17,7 |

### Example 26

3-Bromo-5-trifluoromethylaniline (1,0 mol), tripotassium phosphate (K₃PO₄; 1,2 mol), 4-methylimidazole (1,5 mol) were stirred and heated to 80 °C under argon. 8-Hydroxyquinoline (0,1 mol) and copper (I) iodide (0,1 mol) were added to the mass and heated to 165 °C, and keep at this temperature for 4 hours. 1,44 L of ethanol 96% was added to the hot reaction mixture and allowed to cooled below boiling point. The reaction mixture was filtered, the salts were washed on the filter with 2,4 L of ethanol 96%. Activated charcoal (0,01 kg), concentrated HCI (0,24 L) were added to the combined filtrates, the mass brought to boiling, and filtered hot. The filtrate was cooled to (0-5)°C and the resulting suspension stirred at this temperature for 0,5 hour. The mass was filtered, the substance on the filter was rinsed with 96% ethyl alcohol (1 L) and dried at 90 °C under vacuum (0,2-0,3 bar). 0,67 Mol (67 %) of 5-(4-methyl-1H-imidazol-1-yl)-3-(trifluoromethyl)aniline hydrochloride was obtained.

## Claims

1. A process for preparing 5-(4-methyl-1 *H-*imidazol-1 -yl)-3-(trifluoromethyl)-benzenamine hydrochloride (I) 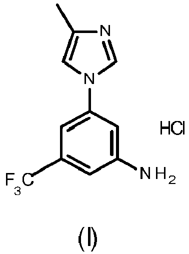 in one-pot reaction comprising the steps of:
a) reacting the compound 3-bromo-5-trifluoromethylaniline with 4-methylimidazole using transition metal catalyst, an additional suitable base in absence of solvent
b) transformation into hydrochloride salt to preparing 5-(4-methyl-1*H-*imidazol-1-yl)-3-(trifluoromethyl)-benzenamine hydrochloride.

2. The process according to Claim 1, wherein the step a) involves transition metal catalysts and ligand.

3. The process according to Claim 2, wherein the transition metal catalyst is palladium.

4. The process according to Claim 2, wherein the transition metal catalyst is copper.

5. The process according to Claim 1 and 2, wherein the acceptable ligands is selected from 8-hydroxyquinoline, 1,3-propanediamine, 1,2-dimethylethylenediamine, *N*, *N*, *N*-trimethyl-1,2-ethanediamine, triethylenetetramine, *trans*-1,2-diaminocyclohexane.

6. The process according to Claim 5, wherein acceptable ligand is 8-hydroxyquinoline.

7. The process according to Claim 1, wherein step a) is performed at a temperature in the range of 160-170°C.
